Europäisches Patentamt

European Patent Office

Office européen des brevets

(11) Publication number: 0 141 362
A2

# EUROPEAN PATENT APPLICATION

(21) Application number: 84112724.4

(22) Date of filing: 22.10.84

(51) Int. Cl.⁴: **C 12 N 15/00**
**C 12 N 1/20**
**//(C12N1/20, C12R1:19)**

(30) Priority: 22.10.83 JP 198043/83

(43) Date of publication of application:
15.05.85 Bulletin 85/20

(84) Designated Contracting States:
BE DE FR GB

(71) Applicant: Tamura, Gakuzo
2-17-12 Sanno Ohta-ku
Tokyo(JP)

(72) Inventor: Yoda, Koji
5-22-15, Mejiro
Toshiam-ku Tokyo(JP)

(72) Inventor: Kikuchi, Yasuhiro
5-23-3, Daita
Setagaya-ku Tokyo(JP)

(72) Inventor: Yamasaki, Makari
2-1-10-4-102, Harumi-cho
Fuchu-shi Tokyo(JP)

(74) Representative: Casalonga, Axel et al,
BUREAU D.A. CASALONGA OFFICE JOSSE & PETIT
Baaderstrasse 12-14
D-8000 München 5(DE)

(54) Novel plasmid vector.

(57) Vector plasmids are constructed to provide a plasmid vector capable of being packaged as a single-stranded phage-like particle by superinfection of helper phage and having suitable restriction sites for easy insertion of a desired gene and further having an advantage that the gene once inserted can be readily rearranged as desired.

EP 0 141 362 A2

./...

Croydon Printing Company Ltd

Fig. 1

## TITLE OF THE INVENTION

## NOVEL PLASMID VECTOR

### Background of the Invention

To express a cloned gene, it is necessary to place it at optimal distances from DNA sequences in expression vectors involved in transcription and translation control. For example, genes must be placed in frame when ligated to the translational initiation codon or signal sequence in a vector.

In the case of an invention relating to the present applicant's prior application (Japanese Patent Application No. 147726/82), a set of three vectors (Psi vectors) having restriction enzyme cleavage sites in the different reading frames is provided. The procedure using these vectors is only applicable to those cases where ligation is done between DNA fragments digested by the restriction enzymes for which the sites exist in the vectors.

To overcome these problems, a process which comprises making cleavage at a restriction enzyme site within a structural gene, removing the sequence uptream of the site corresponding to the N-terminal region, and filling the space between the control signals for expression and the truncated structural gene with a synthetic DNA has been developed and utilized in the production of human growth hormone and $\alpha$-interferon (US Patent No. 4,342,832).

However, the said process requires synthesis of at least two oligonucleotides, and furthermore longer oligonucleotides must be synthesized, as the cleavage site for enzyme is more distant from the site corresponding to the N-terminal amino acid of the desired protein.

As a substitute for the said process, the method of Wallace is available [R. B. Wallace et. al., Science 209, 1396-1400 (1980)], which is known as oligonucleotide-directed site-specific mutagenesis. The said literature discloses a procedure to delete an intervening sequence from cDNA of a yeast tRNA. First, cccDNA (covalently closed circular DNA) of a plasmid is partially digested by a restriction enzyme in the presence of ethidium bromide to bring about nicks, and then single-stranded circular DNA is formed by exonuclease III. With this as a template, a synthetic oligonucleotide of 21-mer complementary to both flanking regions of the part which is desired to be deleted is synthesized, and is used as a primer to form a heteroduplex by _in vitro_ DNA synthesis and ligation. Then, the heteroduplex is used to transform _Escherichia coli_ and the deleted mutant is segregated by replication. The plasmid DNA is extracted from the mixture of these colonies. The mutant plasmid is purified by retransformation, and the strain carrying the mutant plasmid is selected as a clone that can hybridize with the $^{32}P$-labelled primer.

According to the said process, only one oligonucleotide of about 20-mer is necessary as a synthetic DNA. The oligonucleotide can be utilized as a primer or a probe, and there is also an advantage that the deletion is not limited by the availability of restriction enzyme site, etc. at all. However, there is a problem in the preparation of the template DNA in this process. That is, partial decomposition by a restriction enzyme, treatment with exonuclease III and purification of the single-stranded DNA, etc. are complicated and difficult processes, and thus its preparation becomes more difficult with a plasmid of larger size. It is possible to prepare a single-stranded DNA after subcloning to a single-stranded phage vector, but there are troubles of frequent spontaneous deletions and decreases in the burst size of phage when a large DNA fragment is cloned.

As a result of studies to overcome these problems, the present inventors have developed a plasmid vector capable of being packaged as a single-stranded phage-like particle by superinfection of a helper phage and have established the present invention.

Summary of the Invention

The present invention relates to a plasmid vector capable of being packaged as a single-stranded phage-like

- 3 -                                    0141362

particle by superinfection of helper phage.  The novel vector
of the present invention has suitable restriction sites for
easy insertion of a desired gene and further has an advantage
that the gene once inserted can be readily rearranged as
desired.


Brief Description of the Drawings

In the accompanying drawings:

Fig. 1 illustrates a process for constructing
plasmid pYK314.

Fig. 2 illustrates a process for constructing
plasmid pYK330.

Fig. 3 illustrates a process for constructing
plasmids pYK331 and pYK332.

Fig. 4 illustrates a process for constructing
plasmids pYK335 and pYK336, where H represents HindIII; P
represents PstI; Sal represents SalI; X represents XbaI; B
represents BamHI; Sma represents SmaI; Sst represents SstI; E
represents EcoRI, respectively, and MCS represents
multilinker sequence.


Detailed Description of the Invention

The present invention provides a plasmid vector
capable of being packaged as a single-stranded phage-like
particle by superinfection of a helper phage.

The present plasmid vector can be constructed by
integrating into an expression vector a fragment containing a
base sequence necessary for propagation of a single-stranded
filamentous phage (hereinafter referred to as the sfp region
as an abbreviation for single-stranded filamentous
phageness).  The sequence exists in a single-stranded,
filamentous phage DNA and consists of an origin (initiation
point) for replication of C-chain (complementary chain) DNA,
an origin for replication of V-stranded (virus-chain) DNA,
and an origin for packaging.

Any single-stranded filamentous phage can be used,
and, specifically, preferable examples are f1, fd and M13.
These phages are substantially identical with one another, and M13 and fd, and fl and fd are 97% identical with each other in base sequence. The sfp region of these phages, that is, the origin for replication of C-chain DNA, the origin for replication of V-chain DNA, and the origin for packaging, are all in the intergenic region of these phages, and contained in the region of 5489-5868 [Beck & Zink, 1981, Gene 16, 35-38].

Any vector can be used as the expression vector, so long as it contains necessary signals for transcription, translation, secretion of a foreign gene, etc. Furthermore, a vector having a group of restriction enzyme cleavage sites for inserting a desired foreign gene downstream from these signals can be used with more advantage.

As the expression vector, expression vectors containing a promoter of tryptophan operon (Ptrp), a promoter of lactose operon (Plac), a promoter of RecA of Escherichia coli, etc. [Kenzo Nakamura: Kagaku to Seibutsu 20, 47-58 (1982)], and expression and secretion vectors containing a promoter of lipoprotein (Plpp) (the above literature), a promoter of alkaline phosphatase (PphoA) of Escherichia coli [Kikuchi et. al.: Agri. Biol. Chem. 45, 2401-2402 (1981)], etc. can be used.

Specifically, an expression and secretion vector pTA529 containing a phoA promoter (Japanese Patent Application No. 140748/83) is preferable and used in Example, but other vectors can also be used in substantially the same procedure.

As the vector with cleavage sites for restriction enzyme, known expression vectors having the appropriate restriction sites or vectors constructed by introducing a linker into the expression vectors can be used. To ensure insertion of various restriction fragments, it is preferable to utilize a multilinker sequence comprising many restriction sites. As a specifically preferable example, M13mp10 replication type (RF) DNA is used in Example, but other multilinker sequences can be likewise used.

A process for constructing a plasmid vector according to the present invention will be described in detail below:

Introduction of multilinker sequence possessed by M13mpl0RF-DNA right after the signal sequence of pTA529:

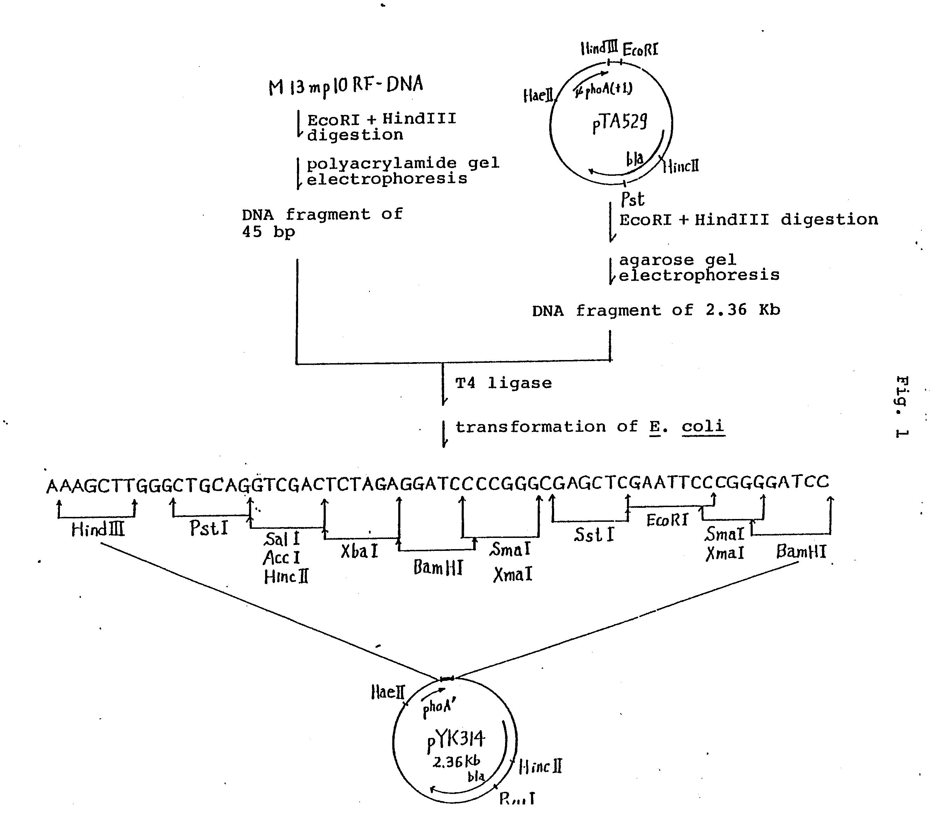

M13mpl0RF-DNA is cleaved by a combination of appropriate restriction enzymes to cut out a multilinker sequence, which is introduced into pTA529. For example, M13mpl0RF-DNA is subjected to double digestion with HindIII and EcoRI to form a fragment of 45 base pairs (bp) containing a multilinker sequence. The fragment is isolated by polyacrylamide gel electrophoresis and linked to a larger fragment [2.36 kilobases (Kb)] of likewise double-digested plasmid pTA529 using T4 ligase, whereby plasmid pYK314 is obtained. Refer to Fig. 1.

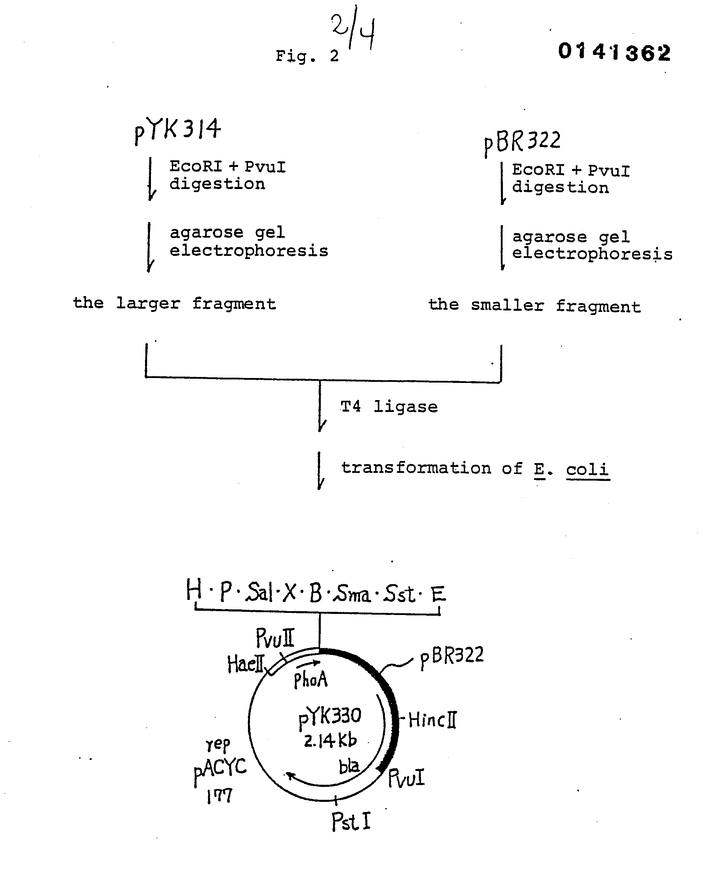

Plasmid pYK314 can be directly used in the following construction process, but before that, a smaller EcoRI-PvuI fragment of pYK314 is replaced with a fragment derived from pBR322 to delete two common restriction sites, whereby plasmid pYK330 is obtained. Refer to Fig. 2.

Introduction of the sfp region of single-stranded phage into pYK330:

The RsaI fragment containing the sfp region of a single-stranded filamentous phage, that is, an origin for replication of C-chain DNA, an origin for replication of V-chain DNA, and an origin for packaging, is inserted into pYK330. These origins exist all together in the region of 5489-5868 in the intergenic region. Since the RsaI fragment of 514bp is a fragment between the sites 5487 and 6001, the whole sfp region is contained in the RsaI fragment.

Insertion of RsaI into pYK330 is carried out by blunt ends or cohesive ends ligation by utilizing an appropriate restriction site of pYK330.

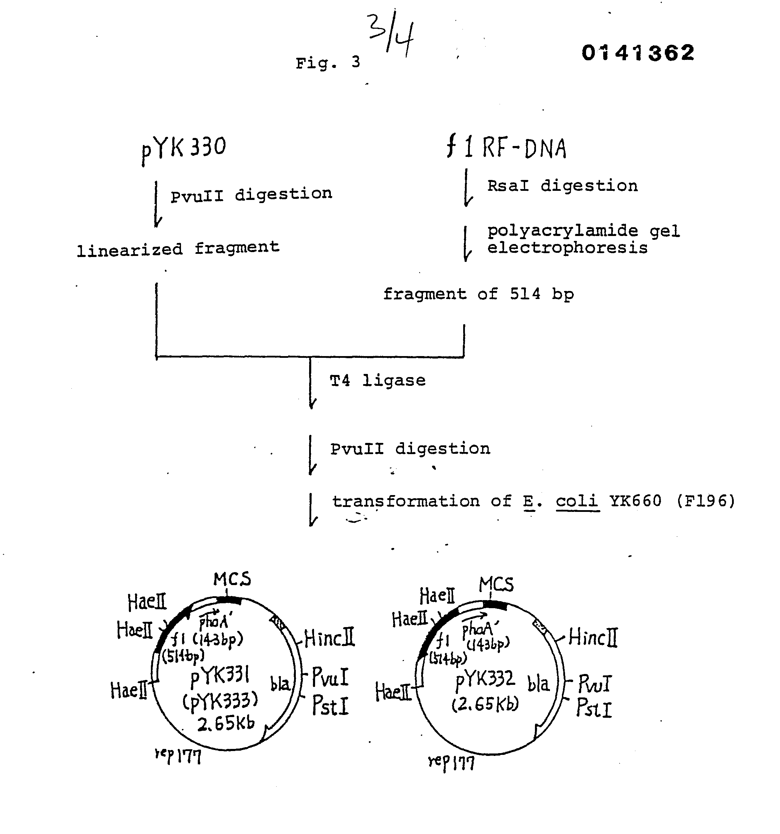

When _Escherichia coli_ YK660 (F196$^+$) is transformed with a recombinant DNA obtained by inserting the RsaI fragment into pYK330, and when the transformants are infected with a wild type fd or fl phage, the desired single-stranded circular pYK331, pYK332 and pYK333 DNAs can be obtained. Refer to Fig. 3.

An assay of pYK331 and pYK332 by agarose gel electrophoresis after treatment with restriction enzymes has revealed that they originate from different plasmid DNA chains and are complementary to each other except some portions. It has been found that pYK331 and pYK333 have the same structure.

pYK331, pYK332 and pYK333 are specific examples of the present plasmid vectors. Plasmid vectors recovered as single-stranded circular DNA after insertion of a desired gene into the three plasmid vectors and successive transformation can be used as templates for the site-specific deletion by appropriate synthesized oligonucleotides to adjust the site of the inserted gene according to the sequences involved in transcription, translation and secretion. Thus, with the present plasmid vectors, rearrangement of a desired gene can be made to make most of various functions such as transcription, translation, secretion, etc. without using a joint of synthesized DNA, etc. The general procedure for rearrangement will be described below:

1.  A recombinant obtained by inserting a DNA fragment containing a desired gene into the multilinker sequence of the present plasmid vector at an appropriate site is introduced into a host microorganism to obtain a transformant. For the primary selection of the transformant, an ampicillin resistance ($Ap^R$), etc. is used.

2.  A recombinant DNA is recovered from the transformant to prepare a restriction enzyme map and make sure of the direction of insertion of the desired gene.

3.  The recombinant DNA is introduced into Escherichia coli $F^+$ by transformation. The transformant is selected by $Ap^R$.

4.  The transformant is infected with a wild type fd (or f1, or M13) phage and the phage particles are propagated.

5.  The phage particles in the supernatant fluid of the culture broth are precipitated with polyethyleneglycol, and after the concentration, protein is removed by phenol extraction to obtain single-stranded DNA.

6.

```
          signal for      the site used       the desired
          expression      for cloning         structural gene
          ─────────→                    ────────────────────→

   vector ────────┬──────────┬─────╱╱╱╱╱╱╱╱╱╱╱╱╱╱╱╱╱╱╱────
                  ┊          ┊     ┊    ┊
                  C          A     B    D
```

The initiation point and end point of the deletion (A and B, respectively in the above diagram) are determined according to the purposes, and an oligonucleotide complementary to the sequences C-A and B-D (more than 6 bp each) flanking the region to be deleted is synthesized and the 5' terminal is phosphorylated with ATP and polynucleotide kinase to obtain a primer.

7.   The said primer is added to a mixture of single-stranded DNAs containing a recombinant carrying the desired gene, and the mixture is heated at 100°C for one minute, and kept standing for cooling.  Then a polymerization buffer together with the four deoxyribonucleotide-3-phosphates, ATP, dithiothreitol, Klenow fragment of *Escherichia coli* DNA polymerase I, and T4 DNA ligase is added, and the mixture is incubated to form a closed double-stranded DNA.

8.   $F^-$ strain of *Escherichia coli* is transformed with the reaction mixture containing the said closed double-stranded DNA, and $Ap^R$ strain are selected.

9.   After mixing appropriate number of transformants, plasmids are extracted according to the rapid method [H. C. Birnboim et. al., Nucleic Acid Research 7 1513 (1979)].  The plasmids are completely digested with an appropriate restriction enzyme which has a restriction site in the deleted part, and used for retransformation to prevent contamination of the original plasmid and enhance the frequency of transformants with deletion.  The transformant is selected by $Ap^R$.

10. Clones with the deletion is detected, separated and purified according to a colony hybridization method, using the said synthesized primer labelled with $^{32}PO_4$ as a probe.

The foregoing operations can be carried out with pYK331, 332 and 333 containing phoA promoter, and can also be carried out with plasmids pYK335 and pYK336 containing both phoA promoter and lac promoter.

pYK335 can be obtained by subjecting pYK331 and plasmid pBH20 containing lac promoter to double digestion with the restriction enzymes EcoRI and PvuI, respectively, and, after separation by agarose gel electrophoresis, ligating by T4 ligase the fragment containing the phoA promoter obtained from the former to the fragment containing the lac promoter from the latter.

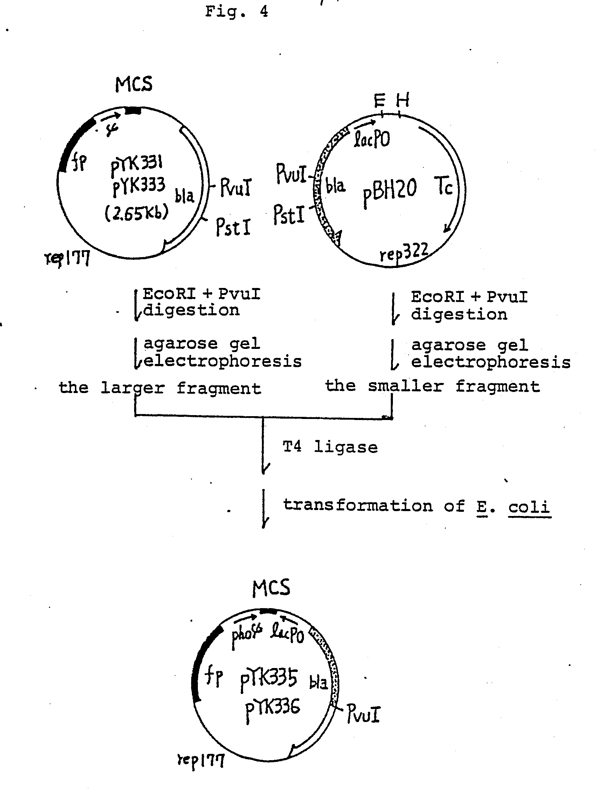

pYK336 can be obtained likewise by using pYK332 in place of pYK331. Construction and the restriction enzyme maps of pYK335 and pYK336 are shown in Fig. 4.

Certain specific embodiments of the invention are described in the following representative example.

Example 1

(1) Introduction of multilinker sequence into ψ-vector:

A multilinker sequence was prepared by subjecting 50 μg of M13mpl0RF-DNA (product of Wako Junyaku Kogyo Co.) to double digestion with the restriction enzymes HindIII and EcoRI (products of Takara Shuzo Co.) and isolating a fragment of 45 base pairs (bp) by polyacrylamide gel electrophoresis. The digestion was carried out at 37°C for one hour in 500 μℓ of a buffer consisting of 7 mM tris (hydroxymethyl) aminomethane (Tris)-HCl (pH 7.4), 7 mM $MgCl_2$, 7 mM 2-mercaptoethanol and 50 mM NaCl and containing 100 units each of the enzymes. Then, 100 μℓ of a stopper solution (pH 7.6) consisting of 50 mM EDTA, 0.1% bromophenol blue (BPB) and 5% Ficoll400 (product of Pharmacia Fine Chemicals) was added thereto, and the mixture was heated at 70°C for 5 minutes to discontinue the reaction. The electrophoresis was carried out with 10% polyacrylamide gel using an electrophoresis buffer containing 40 mM Tris-acetate (pH 7.8) and 1 mM EDTA under an electric current of 20 mA for 15 minutes. After electrophoresis, the gel was stained with 0.5 µg/mℓ ethidium bromide for 10 minutes, and a band slightly above BPB at the front was cut out.  The gel cutout was ground on a glass plate with a flat spatula and then suspended in 5 mℓ of a DNA eluting solution consisting of 500 mM ammonium acetate, 10 mM magnesium acetate, 0.1 mM EDTA and 0.1% sodium dodecylsulfate (SDS) and subjected to extraction with shaking at 37°C for 2 hours.  The supernatant was filtered through a 0.22-µ membrane filter (product of Millipore Co.) and concentrated to about 0.5 mℓ with sec-butanol.  After the concentration, an ethanol solution containing 10 parts (by volume) of ethanol and 1 part of a solution containing 3M sodium acetate, 0.1M magnesium acetate and 1 mM EDTA as auxiliary agents was added thereto, and the mixture was left standing at -40°C for 30 minutes.  Precipitates of the thus formed DNA was washed with 95% ethanol and then with ethyl ether, dried at room temperature and dissolved in 50 µℓ of TES buffer (pH 8.0) consisting of 0.03M Tris, 0.005M EDTA and 0.05M NaCl. The DNA comprises a fragment of 45 bp derived from M13mp10.

Separately, pTA529, one of the ψ vectors, was prepared from Escherichia coli strain containing it (Japanese Patent Application No. 140748/83) according to the method of A. Coleman et. al. [European J. Biochemistry 91, 303-310 (1978)].

Then, 50 µg of pTA529 was subjected to double digestion with EcoRI and HindIII in the same manner as above, and a fragment of 2.36 kilobase pairs (Kb) was isolated by agarose gel electrophoresis.  The agarose gel electrophoresis was carried out with 1.0% agarose gel (20 x 15 x 0.5 cm) under 150 volts for 2 hours [buffer:  40 mM Tris-acetate (pH 7.8)], and staining with 0.5 µg/mℓ ethidium bromide was conducted for 5 - 10 minutes.  After the staining, a portion containing the said fragment of 2.36 Kb was cut out under ultraviolet irradiation, wrapped in a parafilm, and frozen at -40°C for 30 minutes.  The frozen gel was pressed lightly with fingers and the liquid oozed from the gel was collected into an Eppendorf tube through openings of the parafilm.  The liquid was treated with ethanol in the same manner as above, and DNA was recovered as precipitates. The precipitates were treated with ether, dried with air, and dissolved in 0.1 mℓ of TES buffer.

Then, 1 µg of DNA fragment of 45 bp derived from M13mp10 and 20 µg of DNA fragment of 2.3 Kb derived from pYK529 were ligated by T4 ligase, utilizing their common sticky ends.

The ligation reaction was carried out in a mixture of 10 units of T4 ligase (product of New England Biolabs), 20 mM Tris-HCl (pH 7.6), 10 mM $MgCl_2$, 10 mM dithiothreitol, and 0.5 mM ATP at 25°C for one hour. After the reaction, Escherichia coli MC1061 strain (ΔlacZ) [Casadaban et al:, J. Bacteriol., 143 971-980 (1980)] was transformed with the ligation mixture, and ampicillin-resistant ($Ap^R$) strains were selected in a bouillon medium (product of Eiken Kagaku Co.) containing 100 µg/mℓ ampicillin (Ap) . The transformation was carried out according to the method of Mandel-Higa [J. Mol. Biol. , 53, 159 (1970)].

The thus obtained $Ap^R$ strains were treated in the same manner as above to prepare plasmids, and the plasmids were treated with restriction enzymes and analyzed by agarose gel electrophoresis. One of the thus obtained $Ap^R$ strains contained a plasmid (2.36 Kb) of desired structure as shown in Fig. 1 and the plasmid was named pYK314.

(2)   Construction of pYK330 from pYK314:

Plasmid pYK314 had two restriction enzyme SmaI cleavage sites (hereinafter referred to as SmaI sites) and two BamHI sites and the part between the sequence derived from phoA and β-lactamase gene was derived from plasmid pACYC177. Thus, the following operations were conducted to reduce the two cleavage sites for each enzyme to one and to replace the part derived from pACYC177 by the part derived from pBR322.

Plasmid pYK314 was prepared from strains containing it in the same manner as above. The thus obtained pYK314 was subjected to double digestion with EcoRI and PvuI (product of Behringer-Mannheim GmbH.). The digestion was carried out at

- 11 -

0141362

37°C for 60 minutes by dissolving about 10 µg of pYK314 DNA in 200 µℓ of TES buffer, adding 20 µℓ of a solution consisting of 70 mM Tris-HCl (pH 7.4), 70 mM $MgCl_2$, 70 mM 2-mercaptoethanol thereto, adding NaCl thereto to make the concentration 100 mM, and further adding 25 units each of the restriction enzymes thereto. After the reaction, a larger fragment was isolated therefrom by agarose gel electrophoresis in the same manner as above.

Separately, pBR322 [Bolivar et al., Gene, 2, 95 (1977)] was likewise subjected to double digestion, and a smaller fragment was isolated therefrom by agarose gel electrophoresis.

The two fragments derived from pYK314 and pBR322 were ligated by T4 ligase, utilizing the common sticky ends. Escherichia coli MC1061 strain was transformed by the ligation mixture and $Ap^R$ strains were selected.

The foregoing operations were all conducted in the same manner as above. The structures of plasmids carried by the $Ap^R$ strains were investigated in the same manner as above using appropriate restriction enzymes, and a strain having the plasmid of the desired structure was obtained. The plasmid was named pYK330 (refer to Fig. 2). Escherichia coli containing pYK330 was deposited with the Fermentation Research Institute, Agency of Industrial Science and Technology (FERM) as Escherichia coli YK960 (FERM BP-368) on September 26, 1983.

(3) Cloning of single-stranded DNA phage fragment in pYK330:

Plasmid pYK330 was prepared from the strain carrying it in the same manner as above. The thus obtained plasmid was linearized with PvuII (product of Behringer-Mannheim GmbH). The reaction was carried out at 37°C for one hour by reacting about 10 µg of the plasmid with 25 units of the enzyme in 200 µℓ of a solution containing 7 mM Tris-HCl (pH 7.4), 7 mM $MgCl_2$, 100 mM NaCl and 7 mM mercaptoethanol.

Replication form DNA of single-stranded DNA phage fl (flRF DNA) was prepared in the following manner.

Escherichia coli YK660 (F') strain was cultured with shaking in 1ℓ of a bouillon medium at 37°C. When the number of cells reached $10^8$/mℓ, the f1 phage was added at 0.01 - 0.1 m.o.i. (multiplicity of infection) and culturing was continued at 37°C overnight. After the recovery of the cells, phage DNA (f1RF DNA) was prepared in the same manner as in the preparation of plasmid.

Then, f1RF DNA was cleaved with RsaI (product of Nihon Gene Co.), and a DNA fragment of 514 bp was separated by polyacrylamide gel electrophoresis in the same manner as above. The reaction with RsaI was carried out under the following conditions. That is, 25 µg phage DNA was reacted with 50 units RsaI at 37°C for 2 hours in 7 mM Tris-HCl (pH 7.4), 7 mM $MgCl_2$, and 7 mM 2-mercaptoethanol, and the reaction was discontinued in the same manner as above. A fragment of 514 bp was isolated by 5% polyacrylamide gel electrophoresis.

Then, 10 µg of linearized pYK330 was reacted with 1 µg of the said f1RF DNA fragment of 514 bp at 25°C for one hour, using 2.5 units of T4 ligase to link each other through blunt-end ligation. The reaction was discontinued by heating at 70°C for 5 minutes, and those returned to the original pYK330 were destructed by PvuII treatment. Escherichia coli YK660 (F196) was transformed with the ligation mixture, and $Ap^R$ transformants were selected in the same manner as above. The ligation reaction and transformation were carried out in the same manner as above, and the PvuII treatment was carried out at 37°C for 60 minutes by adding 20 units of PvuII to the ligation mixture.

The $Ap^R$ transformants were cultured at 37°C in 1 mℓ of a bouillon medium overnight together with about $10^6$/mℓ of fd wild type viruses, and then the culture broth was subjected to centrifugation at 15,000 rpm for 5 minutes to recover the supernatant.

To the supernatant was added 0.2 mℓ of an aqueous solution consisting of 2.5 M NaCl and 20% polyethyleneglycol (PEG) 6,000, and the mixture was ice-cooled for 30 minutes. Then, the mixture was subjected again to centrifugation at 15,000 rpm for 5 minutes to recover precipitates. The precipitates were suspended in 100 μℓ of TES buffer, and sodium dodecylsulfate (SDS) was added to 0.1%. The mixture was allowed to stand at 25°C for 5 minutes, and then subjected to extraction once with a 10-fold volume of phenol, and twice with 10-fold volume of ether to obtain a single-stranded phage DNA solution. Then, 10 μℓ of the solution was subjected to agarose gel electrophoresis to detect the single-stranded DNA.

Strains showing low molecular weight bands in addition to the single-stranded DNA of fd wild type viruses added as a helper were selected, and the plasmids contained therein were recovered to analyze their structures. The structures of the thus obtained two plasmids are as shown in Fig. 3 as pYK331 and pYK332. pYK331 and pYK332 had the identical structures except that the fragments of fl were inserted in opposite directions. Escherichia coli strains containing pYK331 and pYK332 were deposited with the Fermentation Research Institute as Escherichia coli YK961 (FERM BP-369) and Escherichia coli YK962 (FERM BP-370), respectively, on September 26, 1983.

It was found by the following test that the single-stranded DNAs of pYK331 and pYK332 were derived from different plasmid DNA strands and were complementary to each other.

Five μℓ each of the single-stranded DNAs purified in the same manner as above were together heated at 100°C for one minute in 25 mM Tris-HCl, 7 mM $MgSO_4 \cdot 7H_2O$, 10 mM NaCl, and 7 mM 2-mercaptoethanol, and then the mixture was allowed to stand for cooling and subjected to 0.8% agarose gel electrophoresis. As a result, it was found that bands of larger molecular weight were formed by the heat treatment and cooling of pYK331 and pYK332 together, and it was presumed from their sizes that partial duplex of two DNAs and their multimer were formed.

It is found from the foregoing fact that pYK331 and pYK332 are single-stranded phage-like DNAs each derived from different DNA strands. DNA strands preferable for use will be determined on case-by-case basis considering the sequence of the synthesized DNA necessary for deletion.

(4) Construction of single-stranded DNA vector having a lac promoter:

Ten μg of the said plasmid pYK331 and 10 μg of pBH20 [Science, 198, 1056-1063 (1977)] were independently subjected to double digestion with restriction enzymes EcoRI and PvuI, and a larger fragment was isolated from pYK331 and a smaller fragment from pBH20 by agarose gel electrophoresis. These two fragments were linked together with 2.5 units of T4 ligase, and Escherichia coli YK660 was transformed with the ligation mixture. Ap$^R$ strains were selected in the same manner as above.

The plasmid of the thus obtained Ap$^R$ strains was isolated, and its structure was analyzed by agarose gel electrophoresis, whereby plasmid pYK335 having a structure as in Fig. 4 was identified. pYK335 is an expression vector in which phoA promoter and lac promoter exist on both sides of the multilinker sequence in opposite directions, and a desired gene inserted at the restriction site of the multilinker sequence is under control of phoA promoter or lac promoter, depending on the direction of its insertion.

The site of the desired gene inserted can be adjusted as desired according to the regulatory sequence of expression and secretion by annealing a synthesized DNA that is designed to attain a desired modification (adjustment of reading frame, removal of unnecessary sequence, and optimization of expression and secretion) to the single-stranded DNA with an insertion of the desired gene, synthesizing a double-stranded DNA with the synthesized DNA as a primer and the single-stranded DNA as a template, and selecting the desired clone with the synthesized DNA as a probe.

Plasmid pYK336 in which the sfp was inserted in the opposite direction was obtained in the same manner as above, except that pYK332 was used in place of plasmid pYK331.

Escherichia coli strains containing pYK335 and pYK336 have been deposited with the Fermentation Research

Institute as <u>Escherichia</u> <u>coli</u> YK965 (FERM BP-371) and <u>Escherichia</u> <u>coli</u> YK966 (FERM BP-372), respectively.

<u>WHAT IS CLAIMED IS:</u>

1. A plasmid vector capable of being packaged as a single-stranded phage-like particle by superinfection of helper phage.

2. A plasmid vector which is capable of being packaged as a single-stranded phage-like particle by superinfection of helper phage, and constructed by integrating a fragment containing a base sequence necessary for propagation of a single-stranded filamentous phage (referred to as "sfp" hereinafter) into an expression vector.

3. A plasmid vector according to Claim 2, wherein the single-stranded filamentous phage is fl, fd or M13.

4. A plasmid vector according to Claim 2, wherein the sfp contains an origin for replication of C-chain DNA of the single-stranded filamentous phage, an origin for replication of V-chain DNA of the single-stranded filamentous phage, and an origin for packaging.

5. A plasmid vector according to Claim 2, wherein the expression vector contains a promoter of tryptophan operon, a promoter of lactose operon, a promoter of RecA, a promoter of lipoprotein, or a promoter of alkaline phosphatase.

6. A plasmid vector according to Claim 2, wherein a group of restriction enzyme cleavage sites for further inserting a desired foreign gene exist downstream from the promoter.

7. A process for modifying a recombinant DNA, characterized by rearranging as desired a foreign gene inserted into the plasmid vector defined in Claim 6 according to regulator sequences involved in the expression.

8.  A microorganism containing a plasmid vector capable of being packaged as a single-stranded phage-like particle by superinfection of helper phage.

9. A microorganism according to Claim 8 which is selected from <u>Escherichia coli</u> YK960 (FERM BP - 368), YK961 (FERM BP - 369), YK962 (FERM BP - 370), YK 965 (FERM BP - 371) and YK966 (FERM BP - 372).

M 13 mp 10 RF-DNA

EcoRI + HindIII digestion

polyacrylamide gel electrophoresis

DNA fragment of 45 bp

HindIII EcoRI

HaeII φ phoA(+1)

pTA529

bla HincII

Pst

EcoRI + HindIII digestion

agarose gel electrophoresis

DNA fragment of 2.36 Kb

T4 ligase

transformation of _E. coli_

AAAGCTTGGGCTGCAGGTCGACTCTAGAGGATCCCCGGGCGAGCTCGAATTCCCGGGGATCC

HindIII    PstI    Sal I    XbaI    BamHI    SmaI    Sst I    EcoRI    SmaI    BamHI
                   Acc I             XmaI             XmaI
                   HincII

HaeII    phoA'

pYK314
2.36Kb
bla

HincII

PvuI

Fig. 1

1/4

0141362

Fig. 2 2/4

pYK 330                                    f1 RF-DNA

    │ PvuII digestion                       │ RsaI digestion

                                           │ polyacrylamide gel
linearized fragment                        │ electrophoresis

                          fragment of 514 bp

                    │ T4 ligase

                    │ PvuII digestion

                    │ transformation of E. coli YK660 (F196)

MCS

fp   pYK331
     pYK333   bla  ⟩ PvuI
     (2.65Kb)
                   PstI
rep177

E H

lacPO

PvuI  bla  pBH20  Tc
PstI
              rep322

EcoRI + PvuI
digestion

EcoRI + PvuI
digestion

agarose gel
electrophoresis

agarose gel
electrophoresis

the larger fragment

the smaller fragment

T4 ligase

transformation of E. coli

MCS

phoS lacPO

fp  pYK335  bla
    pYK336       PvuI
rep177